# EUROPEAN PATENT APPLICATION

(11) **EP 4 830 752 A2**
(43) Date of publication of application: **28.10.2026**
(21) Application number: 26197132.9
(22) Date of filing: 26.06.2019
(51) Int. Cl.: A61M 11/04

(54) **AN AEROSOL GENERATING COMPONENT FOR A TOBACCO HEATING DEVICE AND A MOUTHPIECE THEREFOR**

(30) Priority: 29.06.2018 GB 201810738
(62) Document number(s) of the earlier application(s) in accordance with Art. 76 EPC: 19736446.6 / 3 813 579
(71) Applicant: Nicoventures Trading Limited, London WC2R 3LA (GB)
(72) Inventor: HEPWORTH, Richard, London, WC2R 3LA (GB); GOMEZ, Pablo, London, WC2R 3LA (GB); BRANTON, Peter, London, WC2R 3LA (GB)
(74) Representative: Harrison, Philip Mark

(57) **Abstract**

There is described a mouthpiece for an aerosol generating component for a tobacco heating device, the mouthpiece comprising one or more wrappers wherein at least one of the one or more wrappers comprises a sheet material having a density in the range 0.2 g/cm³ to 0.45 g/cm³.

## Description

### Technical Field

The present invention relates to an aerosol generating component for a tobacco heating device, a mouthpiece therefor and to methods of manufacturing the aerosol generating component and the mouthpiece.

### Background

Certain tobacco industry products produce an aerosol during use, which is inhaled by a user. For example, smoking articles such as cigarettes burn tobacco during use to create tobacco smoke. Tobacco heating devices heat an aerosol generating material such as tobacco to form an aerosol by heating, but not burning, the material. Such tobacco industry products commonly include mouthpieces through which the aerosol passes to reach the user's mouth.

### Summary

In accordance with some embodiments described herein, in a first aspect there is provided a mouthpiece for an aerosol generating component for a tobacco heating device, the mouthpiece comprising one or more wrappers, wherein at least one of the one or more wrappers comprises a sheet material having a density in the range 0.2 g/cm³ to 0.45 g/cm³.

At least one of the one or more wrappers according to the first aspect can comprise a sheet material having a density in the range 0.30 g/cm³ to 0.45 g/cm³.

The or each of the one or more wrappers according to the first aspect can have a thickness in the range 20 µm to 100 µm. The or each of the one or more wrappers according to the second aspect can have a thickness in the range 30 µm to 70 µm.

The mouthpiece according to the first aspect can comprise a plurality of wrappers, wherein at least one of the wrappers comprises a porous material.

The mouthpiece according to the first aspect can comprise a body of filter material, wherein at least one of the wrappers partially surrounds the body of filter material, the at least one of the wrappers which partially surrounds the body of filter material comprising a material having a density in the range 0.2 g/cm³ to 0.45 g/cm³.

The mouthpiece according to the first aspect can further comprise a flavour modifying additive disposed within the body of filter material.

The flavour modifying additive can be contained within a breakable capsule.

The mouthpiece according to the first aspect can further comprise ventilation holes formed in the wrappers.

In accordance with some embodiments described herein, in a second aspect there is provided an aerosol generating component for a tobacco heating device, the aerosol generating component comprising a mouthpiece according to the first aspect set out above.

The aerosol generating component according to the second aspect can further comprise an aerosol generating material. The aerosol generating material can comprise tobacco material.

The component of the second aspect above can be substantially cylindrical in shape and can have a circumference in the range 16 mm to 19 mm.

In accordance with some embodiments described herein, in a third aspect there is provided a method of manufacturing a mouthpiece according to the first aspect set out above, the method comprising providing a body of filter material, and wrapping a sheet material having a density in the range 0.2 g/cm³ to 0.45 g/cm³ around the body of filter material.

In accordance with some embodiments described herein, in a fourth aspect there is provided an aerosol generating component for a tobacco heating device, the aerosol generating component comprising an aerosol generating material, a mouthpiece downstream of the aerosol generating material, a first wrapper comprising a first sheet material and surrounding at least a portion of the aerosol generating material, and a second wrapper comprising a second sheet material and surrounding at least a portion of the mouthpiece, wherein the thermal conductivity of the first sheet material is higher than the thermal conductivity of the second sheet material.

The thermal conductivity of the first sheet material can be greater than 0.06 Wm⁻¹K⁻¹.

The thermal conductivity of the second sheet material can be less than 0.06 Wm⁻¹K⁻¹.

The density of the first sheet material can be higher than the density of the second sheet material.

The second wrapper can have a greater thickness than the first wrapper.

In accordance with some embodiments described herein, in a fifth aspect there is provided a method of manufacturing an aerosol generating component for a tobacco heating device, the method comprising, in any order:
wrapping an aerosol generating material in a first wrapper comprising a first sheet material such that the first wrapper surrounds at least a portion of the aerosol generating material;
positioning a mouthpiece downstream of the aerosol generating material; and
wrapping the mouthpiece in a second wrapper comprising a second sheet material such that the second wrapper surrounds at least a portion of the mouthpiece,
wherein the thermal conductivity of the first sheet material is higher than the thermal conductivity of the second sheet material.

### Brief Description of the Drawings

Embodiments of the invention will now be described, by way of example only, with reference to the accompanying drawings, in which:
Figure 1 is a side-on cross sectional view of a tobacco industry product including a mouthpiece;
Figure 2 is a side-on cross sectional view of a further tobacco industry product including a mouthpiece;
Figure 3 is a cross sectional view of the mouthpiece shown in Figure 2;
Figure 4 is a flow diagram illustrating a method of manufacturing an aerosol generating component for a tobacco heating device; and
Figure 5 is a flow diagram illustrating a method of manufacturing a mouthpiece.

### Detailed Description

As used herein, the term "tobacco industry product" is intended to include smoking articles comprising combustible smoking articles and their components such as cigarettes, cigarillos, cigars, tobacco for pipes or for roll-your-own cigarettes, (whether based on tobacco, tobacco derivatives, expanded tobacco, reconstituted tobacco, tobacco substitutes or other smokable material), electronic smoking articles and their components such as e-cigarettes, heating devices that release compounds from substrate materials without burning (where the substrate material can be tobacco, tobacco derivatives, expanded tobacco, reconstituted tobacco, tobacco substitutes or other smokable material), and their components, such as tobacco heating products and components of tobacco heated products including aerosol generating components and mouthpieces for such components, and hybrid systems to generate aerosol from a combination of substrate materials, for example hybrid systems containing a liquid or gel or solid substrate; and aerosol-free nicotine delivery articles and their components such as articles comprising breathable powders. Components of the above articles include consumable smoking article components, mouthpieces, filter units, filter plugs, filter inserts and tubes for use in products such as smoking articles.

The tobacco industry products described herein can be consumable components for use with heating devices, such as aerosol generating components for a tobacco heating device. Alternatively, the tobacco industry products described herein can be smoking articles for combustion, selected from the group consisting of cigarettes, cigarillos and cigars.

Tobacco industry products such as consumable components and smoking articles are often named according to the product length: "regular" (typically in the range 68 - 75 mm, e.g. from about 68 mm to about 72 mm), "short" or "mini" (68 mm or less), "king-size" (typically in the range 75 - 91 mm, e.g. from about 79 mm to about 88 mm), "long" or "super-king" (typically in the range 91 - 105 mm, e.g. from about 94 mm to about 101 mm) and "ultra-long" (typically in the range from about 110 mm to about 121 mm).

They are also named according to the product circumference: "regular" (about 23 - 25 mm), "wide" (greater than 25 mm), "slim" (about 22 - 23 mm), "demi-slim" (about 19 - 22 mm), "super-slim" (about 16 - 19 mm), and "micro-slim" (less than about 16 mm).

Accordingly, a cigarette in a king-size, super-slim format will, for example, have a length of about 83 mm and a circumference of about 17 mm. Cigarettes in the regular, king-size format are preferred by many customers, namely with a circumference of from 23 to 25 mm and an overall length of from 75 to 91 mm.

Each format may be produced with mouthpieces of different lengths, smaller mouthpieces being generally used in formats of smaller lengths and circumferences. Typically the mouthpiece length will be from about 15 mm, associated with short, regular formats, to 30 mm, associated with ultra-long super-slim formats. The tipping paper will have a greater length than the mouthpiece, for example from 3 to 10 mm longer, such that the tipping paper covers the mouthpiece and overlaps the tobacco rod to connect the mouthpiece to the tobacco rod.

Tobacco industry products including smoking articles and their components, aerosol generating components and mouthpieces described herein can be made in, but are not limited to, any of the above formats.

The terms 'upstream' and 'downstream' used herein are relative terms defined in relation to the direction of mainstream aerosol (e.g. smoke) drawn though a tobacco industry product or component in use.

The filter material described herein can comprise cellulose acetate fibre tow. The filter material can also be formed using other materials used to form fibres, such as polyvinyl alcohol (PVOH), polylactic acid (PLA), polycaprolactone (PCL), poly(1-4 butanediol succinate) (PBS), poly(butylene adipate-co-terephthalate)(PBAT), starch based materials, paper, cotton, aliphatic polyester materials and polysaccharide polymers or a combination thereof. The filter material may be plasticised with a suitable plasticiser for the filter material, such as triacetin where the filter material is cellulose acetate tow, or may be non-plasticised. The tow can have any suitable specification, such as fibres having a 'Y' shaped or other cross section, filamentary denier values between 2.5 and 15 denier per filament, for example between 8.0 and 11.0 denier per filament and total denier values of 5,000 to 50,000, for example between 10,000 and 20,000.

As used herein, the term "tobacco material" refers to any material comprising tobacco or derivatives or substitutes thereof. The term "tobacco material" may include one or more of tobacco, tobacco derivatives, expanded tobacco, reconstituted tobacco or tobacco substitutes. The tobacco material may comprise one or more of ground tobacco, tobacco fibre, cut tobacco, extruded tobacco, tobacco stem, reconstituted tobacco and/or tobacco extract.

As used herein, the terms "flavour" and "flavourant" refer to materials which, where local regulations permit, may be used to create a desired taste or aroma in a product for adult consumers. They may include extracts (e.g., liquorice, hydrangea, Japanese white bark magnolia leaf, chamomile, fenugreek, clove, menthol, Japanese mint, aniseed, cinnamon, herb, wintergreen, cherry, berry, peach, apple, Drambuie, bourbon, scotch, whiskey, spearmint, peppermint, lavender, cardamom, celery, cascarilla, nutmeg, sandalwood, bergamot, geranium, honey essence, rose oil, vanilla, lemon oil, orange oil, cassia, caraway, cognac, jasmine, ylang-ylang, sage, fennel, piment, ginger, anise, coriander, coffee, or a mint oil from any species of the genus Mentha), flavour enhancers, bitterness receptor site blockers, sensorial receptor site activators or stimulators, sugars and/or sugar substitutes (e.g., sucralose, acesulfame potassium, aspartame, saccharine, cyclamates, lactose, sucrose, glucose, fructose, sorbitol, or mannitol), and other additives or substances such as charcoal, chlorophyll, minerals, botanicals, or breath freshening agents. They may be imitation, synthetic or natural ingredients or blends thereof. They may be in any suitable form, for example, oil, liquid, or powder.

In the figures described herein, like reference numerals are used to illustrate equivalent features, articles or components.

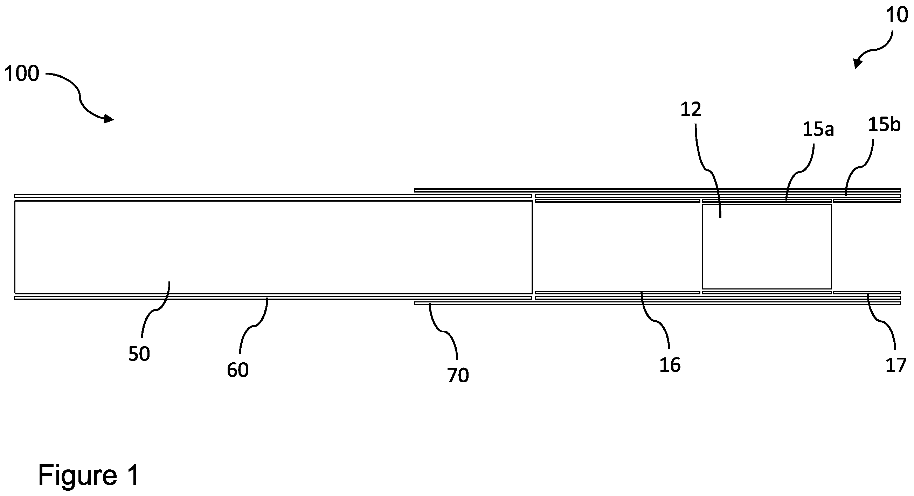

Figure 1 is a side-on cross sectional view of a tobacco industry product, in the present case a consumable component 100 for use with a heating device.

The consumable component 100 comprises a mouthpiece 10, and a cylindrical rod of aerosol generating material 50, in the present case tobacco material, connected to the mouthpiece 10. In the present example, the consumable component 100 has an outer circumference of about 17 mm (i.e. super-slim format).

In other examples, the tobacco industry product may be a smoking article, such as a cigarette in any of the formats described herein. In such examples, the mouthpiece may comprise a filter to filter smoke produced by the smoking article.

The rod of aerosol generating material 50 is wrapped in a first wrapper 60, such that the first wrapper 60 surrounds a lateral surface of the rod of aerosol generating material 50. In the present case, the first wrapper 60 is formed from paper. The first wrapper 60 directly contacts the rod of aerosol generating material 50. The first wrapper 60, in the present example, has a density of 0.60 g/cm³, being formed from material having a thickness of 40 µm and a basis weight of 24 g/m². In alternative examples, the first wrapper 60 can have a density in the range 0.5 to 1.0 g/cm³, for instance from 0.50 to 0.70 g/cm³. The rod of aerosol generating material 50 is 42 mm in length, although it can be any length from 20 mm to 80 mm, for instance from 30 mm to 60 mm, or from 35 mm to 55 mm. A connecting wrapper 70, in the present case formed from paper, overlies the first wrapper 60 and connects the mouthpiece 10 to the rod of aerosol generating material 50.

The consumable component 10 comprises one or more second wrappers, in the present case an inner wrapper 15a and an outer wrapper 15b, each at least partially surrounding the mouthpiece 10. Each of the inner wrapper 15a and the outer wrapper 15b is formed from a sheet material, in the present case paper. In the present example, the density of the outer second wrapper 15b is 0.38 g/cm³. The outer second wrapper 15b has a thickness of 60 µm and a basis weight of 23 g/m². At least one of the inner and outer second wrappers 15a, 15b can comprise a sheet material having a density in the range 0.2 to 0.45 g/cm³. The sheet material forming at least one of the inner and outer second wrappers 15a, 15b can have a density in the range 0.30 g/cm³ to 0.45 g/cm³, or 0.35 g/cm³ to 0.45 g/cm³.

The sheet material forming the first wrapper 60 has a higher thermal conductivity than that of the sheet material forming one or each of the second wrappers 15a, 15b. This can be achieved by selecting appropriate values for the density of the first and second wrappers 60, 15a, 15b. The thermal conductivity of the sheet material forming the first wrapper 60 is 0.07 Wm⁻¹K⁻¹. In other examples, the thermal conductivity of the sheet material forming the first wrapper 60 can be greater than 0.06 Wm⁻¹K⁻¹. The thermal conductivity of the sheet material forming the first wrapper 60 can, for example, be between 0.06 Wm⁻¹K⁻¹ and 1.2 Wm⁻¹K⁻¹, for instance 0.08 to 1.1 Wm⁻¹K⁻¹. Thermal conductivities set out herein are those of the materials forming the relevant sheet wrappers, irrespective of the thickness of the wrappers.

The thermal conductivity of the sheet material forming the outer second wrapper 15b is 0.04 Wm⁻¹K⁻¹, and the thermal conductivity of the sheet material forming the inner second wrapper 15a is 0.07 Wm⁻¹K⁻¹. In other examples, the thermal conductivity of the sheet material forming either or both of the inner and outer second wrappers 15a, 15b, is less than 0.06 Wm⁻¹K⁻¹, for example between 0.02 Wm⁻¹K⁻¹ and 0.06 Wm⁻¹K⁻¹.

Thermal conductivity can be measured using a known technique, such as using the Model C-2500-HTS Thermal Conductivity system available from International Thermal Instrument Company, Inc., in Del Mar, California, US. Calibration of the apparatus and performance of the testing can follow the current ASTM C177 standard.

Although in the present example the mouthpiece 10 is described as comprising two second wrappers 15a, 15b, the number of second wrappers is not limited thereto. The mouthpiece 10 may comprise three or more second wrappers, or alternatively may comprise a single second wrapper, whether that is the inner second wrapper 15a or the outer second wrapper 15b. In the case that there are three second wrappers, an additional middle wrapper can be provided between the inner and outer second wrappers 15a, 15b. The outermost of the three second wrappers 15b at least can be arranged to have the low density and/or low thermal conductivity features defined herein.

The connecting wrapper 70 can alternatively be the second wrapper, or can be one of a plurality of second wrappers. In particular, the density of the material forming the connecting wrapper 70 can be 0.38 g/cm³. The connecting wrapper 70 can have a thickness of about 60 µm and a basis weight of about 23 g/m². Alternatively, the density of the material forming the connecting wrapper 70 can be in the range 0.2 to 0.45 g/cm³, for instance in the range 0.30 g/cm³ to 0.45 g/cm³, or 0.35 g/cm³ to 0.45 g/cm³. The thermal conductivity of the sheet material forming the connecting wrapper 70 can be 0.04 Wm⁻¹K⁻¹. In other examples, the thermal conductivity of the connecting wrapper 70 is less than 0.06 Wm⁻¹K⁻¹, for example between 0.02 Wm⁻¹K⁻¹ and 0.06 Wm⁻¹K⁻¹.

In use, the component 100 can be inserted into a heating device (not shown) such that at least a portion of the mouthpiece 10 protrudes from the heating device and can be inserted into a user's mouth. An aerosol is produced by heating the aerosol generating material 50 using the heating device. The aerosol produced by the aerosol generating material 50 passes through the mouthpiece 10 to the user's mouth.

Since the sheet material forming the first wrapper 60 has a higher thermal conductivity than that of the sheet material forming one or both of the second wrappers 15a, 15b, heat transfer into, out of and within the component 100 can be improved compared designs of components where similar thermal conductivity is exhibited by the first and second wrappers. The arrangement of the components described herein leads to more heat being conducted through each unit thickness of the first wrapper 60 and into the aerosol generating material 50 than through each unit thickness of the second wrapper(s) at least partially surrounding the mouthpiece 10. This arrangement can allow for good thermal conduction into the rod of aerosol generating material 50 during use, to facilitate the generation of an aerosol, while reducing thermal conduction through one or both of the second wrappers 15a, 15b, reducing the temperature of components which come into contact with the mouth or fingers of a user.

One or both of the second wrappers 15a, 15b can have a greater thickness than the first wrapper 60. For instance, the thickness of one or both of the second wrappers 15a, 15b can be between 42 µm and 80 µm, for instance between 42 µm and 70 µm or between 50 µm and 70 µm. The thickness of the first wrapper 60 can be between 20 and 50 µm, for instance between 30 and 45 µm.

Alternatively or in addition, the density of the sheet material forming the first wrapper 60 may be higher than the density of the sheet material forming one or both of the second wrappers 15a, 15b. Again, this aids thermal conduction through the first wrapper 60 compared to the thermal conduction through one or both of the second wrappers 15a, 15b.

As an example, providing a second wrapper comprising a sheet material having a density in the range 0.2 g/cm³ to 0.45 g/cm³ provides reduced thermal conduction through the wrapper. The sheet material forming the first wrapper 60 may have a density of 0.50 g/cm³ to 0.70 g/cm³.

In some examples, one or both of the second wrappers 15a, 15b can have a thickness in the range 20 µm to 100 µm, or in the range 30 µm to 70 µm. For example, one or each of the second wrappers 15a, 15b may have a thickness of approximately 60 µm. For instance, the outer second wrapper may have a thickness of approximately 60 µm. The inner second wrapper may have a thickness of approximately 40 µm.

One or both of the second wrappers 15a, 15b as described herein (or the single second wrapper, where applicable) can comprise a porous sheet material. A porous material in this context means a material having a permeability greater than 200 Coresta units. The porous sheet material of the second wrapper(s) can have a permeability greater than 500, 1000, 1500 or 2000 Coresta Units (CU). The porous sheet material of the second wrapper(s) can have a permeability between 2000 and 8000 CU. The porous sheet material of the second wrapper(s) preferably has a porosity greater than 2500 CU. Paper can be used as such a sheet material. Alternatively, one or both of the second wrappers 15a, 15b as described herein (or the single second wrapper, where applicable) can comprise a non-porous sheet material, having a permeability less than 200 CU. In this case, a barrier coating or similar modification to the material can be used to provide the correct level of permeability, for instance while retaining desired density and/or thermal conductivity values.

The first wrapper 60 as described herein can comprise a non-porous sheet material. A non-porous material in this context means a material having a permeability of less than 200 CU. The non-porous sheet material of the first wrapper can have a permeability less than 150, 125 or 100 CU. The sheet material of the first wrapper can have a permeability of, for instance, 75 CU.

Tables 1 and 2 below sets out various examples of the wrapper configurations which can be used in the consumable components described herein, and the associated density (Table 1) and thermal conductivity (Table 2) of the wrappers.

**Table 1**

| **Density:** | | | | | | |
|---|---|---|---|---|---|---|
| Example | First Wrapper | | Inner Second Wrapper | Middle (optional) Second Wrapper | Outer Second Wrapper | Connecting Wrapper |
| 1 | 0.60 g/cm³ | | 0.65 g/cm³ | 0.58 g/cm³ | 0.38 g/cm³ | 0.70 g/cm³ |
| 2 | 0.60 g/cm³ | | 0.65 g/cm³ | 0.58 g/cm³ | 0.58 g/cm³ | 0.38 g/cm³ |
| 3 | 0.60 g/cm³ | | 0.65 g/cm³ | None | 0.38 g/cm³ | 0.70 g/cm³ |
| 4 | 0.60 g/cm³ | | 0.38 g/cm³ | None | 0.58 g/cm³ | 0.70 g/cm³ |
| 5 | 0.60 g/cm³ | | 0.65 g/cm³ | None | 0.65 g/cm³ | 0.38 g/cm³ |

**Table 2**

| **Thermal Conductivity:** | | | | | |
|---|---|---|---|---|---|
| Example | First Wrapper | Inner Second Wrapper | Middle (optional) Second Wrapper | Outer Second Wrapper | Connecting Wrapper |
| 6 | 0.06 Wm⁻¹K⁻¹ | 0.07 Wm⁻¹K⁻¹ | 0.06 Wm⁻¹K⁻¹ | 0.04 Wm⁻¹K⁻¹ | 0.08 Wm⁻¹K⁻¹ |
| 7 | 0.06 Wm⁻¹K⁻¹ | 0.07 Wm⁻¹K⁻¹ | 0.06 Wm⁻¹K⁻¹ | 0.06 Wm⁻¹K⁻¹ | 0.04 Wm⁻¹K⁻¹ |
| 8 | 0.06 Wm⁻¹K⁻¹ | 0.07 Wm⁻¹K⁻¹ | None | 0.04 Wm⁻¹K⁻¹ | 0.08 Wm⁻¹K⁻¹ |
| 9 | 0.06 Wm⁻¹K⁻¹ | 0.04 Wm⁻¹K⁻¹ | None | 0.06 Wm⁻¹K⁻¹ | 0.08 Wm⁻¹K⁻¹ |
| 10 | 0.06 Wm⁻¹K⁻¹ | 0.07 Wm⁻¹K⁻¹ | None | 0.07 Wm⁻¹K⁻¹ | 0.04 Wm⁻¹K⁻¹ |

The mouthpiece 10 further comprises a cylindrical body of filter material 12, in the present example cellulose acetate tow. The body of filter material 12 may be approximately 8 mm to 14 mm in length. In the present example, the body of filter material 12 is 10 mm in length. The tow has a denier per filament (d.p.f.) of 9.5 and a total denier of 12,000. The second wrappers 15a, 15b partially surround the body of filter material 12.

The mouthpiece 10 further comprises a first tube segment 16 and a second tube segment 17. The first tube segment 16 is disposed upstream of the body of filter material 12, while the second tube segment 17 is disposed downstream of the body of filter material 12. The second tube segment 17 forms a recess at the mouth end of the mouthpiece 10. The outer second wrapper 15b connects the inner second wrapper 15a with the first tube segment 16 and the second tube segment 17. In the present example, the first tube segment 16 is 25 mm in length, and the second tube segment 17 is 6 mm in length. In other examples, one or both of the tube segments may be omitted.

In the present example, the first and second tube segments 16, 17 are formed from one or more layers of stiff paper. In other examples, one or both of the tube segments may be formed from filter material, e.g. cellulose acetate.

In the present example, ventilation holes (not shown) are formed in the tipping material 70, the outer second wrapper 15b and the first tube segment 16. This arrangement provides ventilation into the mouthpiece 10 at a position upstream of the body of filter material 12. The ventilation holes may be formed in the above components by laser perforation or mechanical perforation.

The ventilation holes may be located between about 17 mm and about 25 mm from the mouth end of the consumable 100. In the present example, the ventilation holes are located about 18 mm from the mouth end of the consumable 100. In other examples, the ventilation holes may be formed in the tipping material 70, the outer second wrapper 15b and the second tube segment 17, so as to provide ventilation into the mouthpiece at a position downstream of the body of filter material 12.

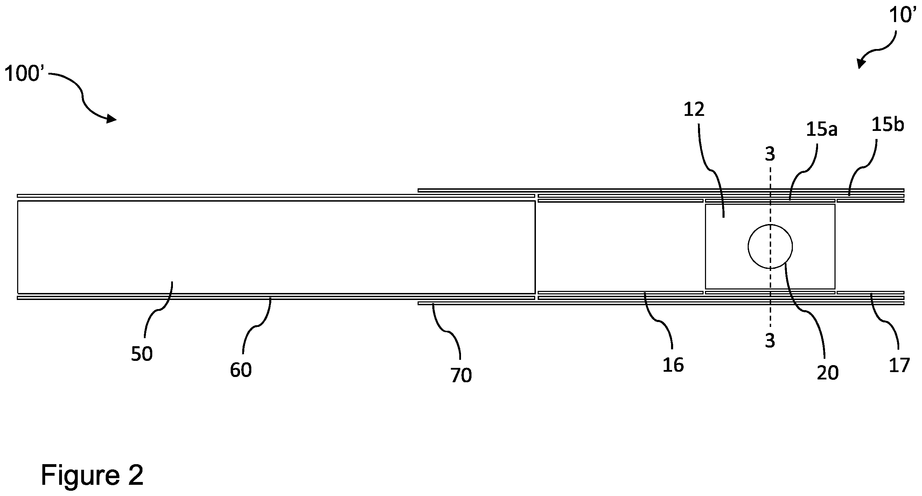

Figure 2 is a side-on cross sectional view of a further tobacco industry product. The further tobacco industry product is a modified consumable component 100' which is substantially the same as the consumable component 100 described above in relation to Figure 1. The differences are described below.

The modified consumable component 100' includes a mouthpiece 10'. The mouthpiece 10' comprises a flavour modifying additive disposed within a body of filter material 12. In the present example, the flavour modifying additive is contained within a breakable capsule 20. In other examples, the flavour modifying additive can be provided as a thread carrying a flavourant, or other aerosol modifying agent may be disposed within the body of filter material 12.

In the present example, a single capsule 20 is used. The capsule 20 is entirely embedded within the filter material 12. In other words, the capsule 20 is completely surrounded by the filter material 12. In other examples, a plurality of breakable capsules may be disposed within the filter material 12, for instance 2, 3 or more breakable capsules. In examples where a plurality of capsules is used, the individual capsules may be the same as each other, or may differ from one another in terms of size and/or capsule payload.

The capsule 20 has a core-shell structure. In other words, the capsule 20 comprises a shell encapsulating a liquid flavourant, which can be any one of the flavourants described herein. The shell of the capsule can be ruptured by a user to release the flavourant into the filter material 12. Forming at least one of the second wrappers 15a, 15b from a non-porous material can act to prevent the liquid flavourant from leaking through the wrapper when the capsule 20 is broken. For instance, the inner second wrapper 15a can comprise a barrier coating to make the material substantially impermeable to the liquid flavourant. Alternatively or in addition, the outer second wrapper 15b can comprise a barrier coating to make the material substantially impermeable to the liquid flavourant.

In use, the user may remove the consumable component from the heating device mid-use to break the capsule 20. Using a second wrapper 15a, 15b providing reduced thermal conduction, as described above, reduces exposure of the user to heat when gripping the wrapper to remove the consumable component and to break the capsule 20.

Providing a second wrapper surrounding the mouthpiece with a density in the ranges described herein also presents a softer surface against which the user has to push to break the capsule.

In the present example, the capsule 20 is spherical and has a diameter of about 3 mm. In other examples, other shapes and sizes of capsule can be used. The total weight of the capsule may be in the range about 10 mg to about 50 mg.

In the present example, the capsule 20 is located at a longitudinally central position within the body of filter material 12. That is, the capsule 20 is positioned so that its centre is 5 mm from each end of the body of filter material 12. In other examples, the capsule 20 can be located at a position other than a longitudinally central position in the body of filter material, i.e. closer to the downstream end of the body of filter material than the upstream end, or closer to the upstream end of the body of filter material than the downstream end. Preferably, mouthpiece 10' is configured so that the capsule 20 and the ventilation holes are longitudinally offset from each other in the mouthpiece 10'.

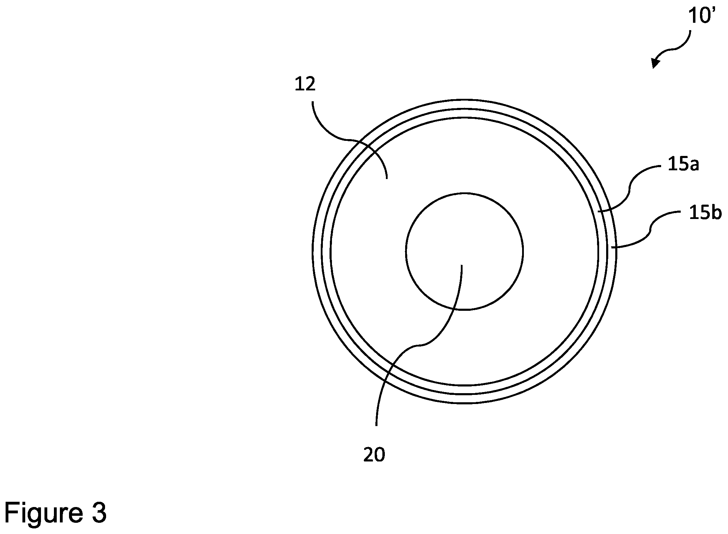

A cross section of the mouthpiece 10' is shown in Figure 3. This figure shows the capsule 20, the body of filter material 12 and the wrappers 15a, 15b. In the present example, the capsule 20 is centred on the longitudinal axis (not shown) of the mouthpiece 10'. The wrappers 15a, 15b are arranged concentrically around the cylindrical body of filter material 12.

In the examples described above, a body of filter material is disposed towards the mouth end of the consumable. Alternatively or additionally, a body of filter material may be disposed at the opposite (distal) end of the consumable.

In the examples described above, the mouthpiece comprises a single body of filter material. In other examples, the mouthpiece may comprise multiple bodies of filter material. The mouthpiece may comprise a cavity between the bodies of filter material.

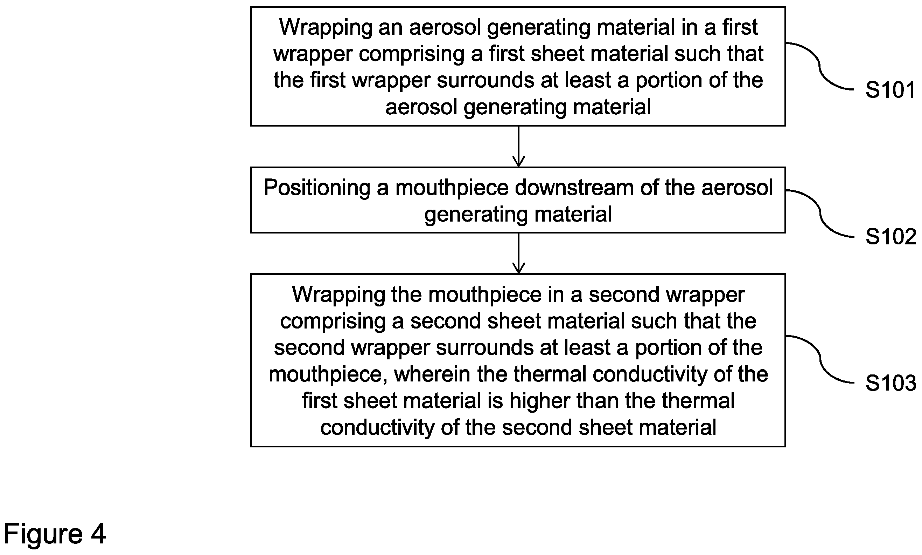

Also provided herein is a method of manufacturing an aerosol generating component for a tobacco heating device. The method is shown in Figure 4 and comprises, in any order: wrapping an aerosol generating material in a first sheet wrapper such that the first sheet wrapper surrounds at least a portion of the aerosol generating material (S101); positioning a mouthpiece downstream of the aerosol generating material (S102); and wrapping the mouthpiece in a second sheet wrapper such that the second sheet wrapper surrounds at least a portion of the mouthpiece, wherein the thermal conductivity of the first sheet wrapper is higher than the thermal conductivity per unit thickness of the second sheet wrapper (S103). S102 and S103 can alternatively be performed in reverse order, such that the mouthpiece is wrapped in the second sheet wrapper before being positioned downstream of the aerosol generating materials.

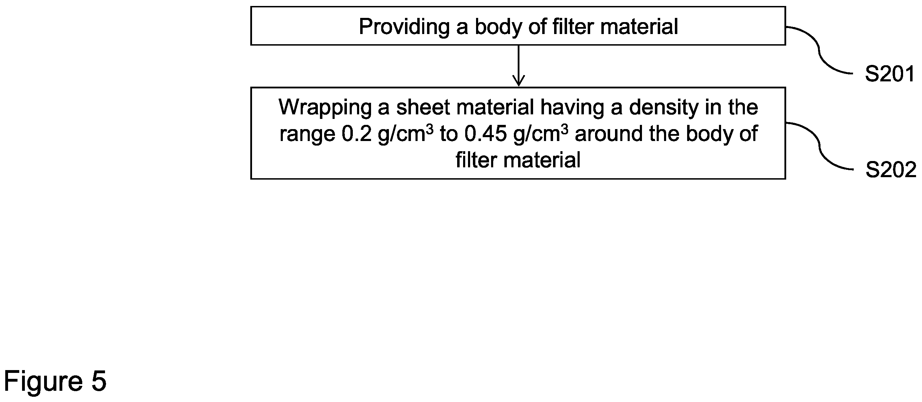

Also presented herein is a method of manufacturing a mouthpiece. The method is shown in Figure 5 and comprises: providing a body of filter material (S201); and wrapping a sheet material having a density in the range 0.2 g/cm³ to 0.45 g/cm³ around the body of filter material (S202).

In order to address various issues and advance the art, the entirety of this disclosure shows by way of illustration various embodiments in which the claimed invention(s) may be practiced and provide for superior aerosol generating components. The advantages and features of the disclosure are of a representative sample of embodiments only, and are not exhaustive and/or exclusive. They are presented only to assist in understanding and teach the claimed features. It is to be understood that advantages, embodiments, examples, functions, features, structures, and/or other aspects of the disclosure are not to be considered limitations on the disclosure as defined by the claims or limitations on equivalents to the claims, and that other embodiments may be utilised and modifications may be made without departing from the scope and/or spirit of the disclosure. Various embodiments may suitably comprise, consist of, or consist essentially of, various combinations of the disclosed elements, components, features, parts, steps, means, etc. In addition, the disclosure includes other inventions not presently claimed, but which may be claimed in future.

## Claims

1. A mouthpiece for an aerosol generating component for a tobacco heating device, the mouthpiece comprising:
one or more wrappers;
wherein at least one of the one or more wrappers comprises a sheet material having a density in the range 0.2 g/cm³ to 0.45 g/cm³.

2. A mouthpiece according to claim 1, wherein at least one of the one or more wrappers comprises a sheet material having a density in the range 0.30 g/cm³ to 0.45 g/cm³.

3. A mouthpiece according to claim 1 or 2, wherein the or each of the one or more wrappers has a thickness in the range 20 µm to 100 µm.

4. A mouthpiece according to claim 3, wherein the or each of the one or more wrappers has a thickness in the range 30 µm to 70 µm.

5. A mouthpiece according to any one of claims 1 to 4, comprising a plurality of wrappers, wherein at least one of the wrappers comprises a porous material.

6. A mouthpiece according to any one of claims 1 to 5, comprising a body of filter material, wherein at least one of the wrappers partially surrounds the body of filter material, the at least one of the wrappers which partially surrounds the body of filter material comprising a material having a density in the range 0.2 g/cm³ to 0.45 g/cm³.

7. A mouthpiece according to claim 6, further comprising a flavour modifying additive disposed within the body of filter material.

8. A mouthpiece according to claim 7, wherein the flavour modifying additive is contained within a breakable capsule.

9. A mouthpiece according to any one of claims 6 to 8, comprising ventilation holes formed in the wrappers.

10. An aerosol generating component for a tobacco heating device, the aerosol generating component comprising a mouthpiece according to any one of claims 1 to 9.

11. An aerosol generating component according to claim 10, further comprising an aerosol generating material.

12. An aerosol generating component according to claim 11, wherein the aerosol generating material comprises tobacco material.

13. An aerosol generating component according to any one of claims 10 to 12, wherein the component is substantially cylindrical in shape and has a circumference in the range 16 mm to 19 mm.

14. A method of manufacturing a mouthpiece according to any one of claims 6 to 9, the method comprising:
providing a body of filter material;
wrapping a sheet material having a density in the range 0.2 g/cm³ to 0.45 g/cm³ around the body of filter material.
